# EUROPEAN PATENT APPLICATION

(11) **EP 2 338 538 A1**
(43) Date of publication of application: **29.06.2011**
(21) Application number: 11000222.7
(22) Date of filing: 10.11.2003
(51) Int. Cl.: A61L 31/16, A61F 2/06, A61L 29/16, A61L 31/10

(54) **Method and apparatus for reducing tissue damage after ischemic injury**

(30) Priority: 08.11.2002 US 425096 P
(62) Divisional of application: 03781881.2
(71) Applicant: Conor Medsystems, Inc., Menlo Park, CA 94061 (US); Shanley, John F., Emerald Hills CA 94062-4072 (US)
(72) Inventor: Litvack, Frank, Los Angeles CA 90010 (US); Parker, Theodore, L., Danville CA 94506 (US); Shanley, John, F., Emerald Hills CA 94062-4072 (US)
(74) Representative: Diehl & Partner GbR

(57) **Abstract**

A method and apparatus for the local delivery of therapeutic agents reduces myocardial tissue damage due to ischemia. A local delivery device is used for delivery of the therapeutic agents into a coronary artery which feeds the ischemic myocardial tissue. According to one example, an implantable medical device for delivering insulin locally to myocardial tissue includes a therapeutic dosage of insulin in a biocompatible polymer affixed to a stent. The therapeutic dosage of insulin is released from the stent at a therapeutic dosage and over an administration period effective to reduce ischemic injury of the myocardial tissue. The therapeutic agents for use in the method are delivered locally to reduce myocardial tissue damage due to ischemia.

## Description

### Cross Reference to Related Applications

This application claims priority to U.S. Provisional Application Serial No. 60/425,096 filed November 8, 2002, which is incorporated herein by reference in its entirety.

### Background

The reduction or cessation of blood flow to a vascular bed accounts for a variety of clinical events that require immediate intervention and restitution of adequate perfusion to the jeopardized organ or tissue. Different tissues can withstand differing degrees of ischemic injury. However, tissues may progress to irreversible injury and cellular necrosis if not reperfused.

Impaired perfusion of cardiac tissue (ischemia) results in a loss of the heart's ability to function properly as the tissue becomes oxygen and energy deprived. Permanent injury is directly related to the duration of the oxygen deficit the myocardium experiences. Ischemia occurs when blood flow to an area of cells is insufficient to support normal metabolic activity. Surgical and percutaneous revascularization techniques following acute myocardial infarction (MI) are highly effective at treating ischemic myocardial tissue. In the case of an acute MI, the main blood flow is stopped by the blockage of a coronary artery and the tissue is perfused only through collateral arteries. If the ischemic condition persists for an extended period, the damage to cells within the ischemic zone progresses to irreversible injury and cellular necrosis. Reperfusion is the term used to describe the act of reestablishing blood flow and oxygen supply to ischemic tissue. Reperfusion is essential to the future survival of cells within an ischemic area. Reperfusion may be achieved by a blood flow recanalization therapy, generally including one of coronary angioplasty, administration of a thrombolytic drug, coronary artery bypass surgery, or the like.

Timely reperfusion of ischemic myocardium limits infarct size and early reperfusion with angioplasty or thrombolytic therapy provides benefits of reduced myocardial damage, improved ventricular function, and reduced mortality in patients with acute MI. Myocardial salvage can however be compromised by such complications as coronary reocclusion and severe residual coronary stenosis.

Reperfusion of the ischemic myocardium does not alone return full functioning of the myocardium. In fact, it is well known that reperfusion itself can cause damage to many cells that survived the ischemic event. Studies have shown that reperfusion may accelerate death of irreversibly injured myocardium, and may also compromise survival of jeopardized, but still viable myocytes salvaged by reperfusion. These so-called reperfusion injuries may represent more than 50% of ultimate infarct size. A number of cellular mechanisms are believed to be responsible for ischemia-induced reperfusion injury. Development of adjuvant treatments to protect the post-ischemic myocardium and maximize benefits of coronary reperfusion has thus become a major target of modem cardiovascular research.

Compounds capable of minimizing and containing ischemic or reperfusion damage represent important therapeutic agents. In the past years, it has been demonstrated that the mortality rates following myocardial infarction and reperfusion can be further improved by delivery of drugs which optimize energy transfer in the post-ischemic heart tissue. For example, an arterial infusion of a combination of glucose, insulin, and potassium (GIK) after an acute myocardial infarction and reperfusion has been shown to provide an impact on the injured but viable myocardium tissue and reduced mortality.

The high level of insulin created by the arterial infusion of GIK has been shown to improve ischemic and post-ischemic myocardial systolic and diastolic function as well as improving coronary vasodilatation. The provision of insulin also preserves and restores myocardial glycogen stores. GIK also decreases circulating levels of arterial free fatty acids (FFAs) and myocardial FFA uptake. High FFA levels are toxic to ischemic myocardium and are associated with increased membrane damage, arrhythmias, and decreased cardiac function. Thus, there are many mechanism by which insulin can reduce ischemic injury. However, when insulin is delivered systemically by arterial infusion, the insulin stimulates glucose and potassium uptake throughout the body and thus reduces glucose and potassium levels in the blood to unsafe levels resulting in hypoglycemia and hypokolemia. GIK therapy thus involves administration of glucose and potassium along with the insulin to mitigate the undesirable systemic side effects of systemic insulin administration and requires careful monitoring of glucose and potassium levels.

In general, the compounds which have been used for reducing tissue damage after acute myocardial infarction have been delivered systemically, such as by arterial infusion. Systemic delivery of these compounds have significant drawbacks including the requirement for additional administration of protective agents to prevent damage to non-target tissues caused by the systemic delivery, i.e. requirement for delivery of glucose and potassium with an insulin infusion. Other drawbacks include the requirement for continuous administration and supervision, suboptimal delivery to the ischemic area, patient discomfort, high dosages required for systemic delivery, and side effects of the systemic delivery and high dosages.

### Summary of the Invention

The present invention relates to the local delivery of therapeutic agents which reduce myocardial tissue damage due to ischemia. The therapeutic agents are delivered locally to the myocardial tissue and over an administration period sufficient to achieve reduction in ischemic injury of the myocardial tissue.

In accordance with one aspect of the invention, a method for reducing tissue damage following ischemic injury includes identifying an implantation site within a blood vessel; delivering an expandable medical device containing a drug which preserves myocardial cell viability into the blood vessel to the selected implantation site; implanting the medical device at the implantation site; and locally delivering a therapeutic agent from the expandable medical device to tissue at the implantation site and to the blood vessels downstream of the implantation site over an administration period sufficient to reduce ischemic injury of the surrounding myocardial cells.

In accordance with another aspect of the invention, a method of delivering insulin locally to myocardial tissue to reduce tissue damage following myocardial infarction and reperfusion includes identifying an occlusion site within a blood vessel; treating the occlusion site to achieve reperfusion; and locally delivering insulin to the tissue at or near the treated occlusion site and downstream of the occlusion site to reduce ischemic injury.

In accordance with an additional aspect of the invention, an implantable medical device for delivering insulin locally to myocardial tissue inclues an implantable medical device configured to be implanted within a coronary artery and a therapeutic dosage of insulin in a biocompatible polymer affixed to the implantable medical device, wherein the therapeutic dosage of insulin is released to the myocardial tissue at a therapeutic dosage and over an administration period effective to reduce ischemic injury of the myocardial tissue.

In accordance with a further aspect of the invention, an implantable medical device for delivering a therapeutic agent locally to myocardial tissue includes an implantable medical device configured to be implanted within a coronary artery, and a therapeutic dosage of a therapeutic agent for treatment of ischemic injury following acute myocardial infarction. The therapeutic agent is affixed to the implantable medical device in a manner such that the therapeutic agent is released to the myocardial tissue at a therapeutic dosage and over an administration period effective to reduce ischemic injury of the myocardial tissue.

In accordance with another aspect of the invention, a stent for delivering insulin locally to myocardial tissue includes a substantially cylindrical expandable device body configured to be implanted within a blood vessel, and a therapeutic dosage of insulin in a biocompatible polymer affixed to the implantable medical device body.

### Brief Description of the Drawings

The invention will now be described in greater detail with reference to the preferred embodiments illustrated in the accompanying drawings, in which like elements bear like reference numerals, and wherein:

FIG. 1 is a cross-sectional perspective view of a portion of an expandable medical device implanted in the lumen of an artery with a therapeutic agent arranged for delivery to the lumen of the artery;

FIG. 2 is a perspective view of an expandable medical device showing a plurality of openings;

FIG. 3 is an expanded side view of a portion of the expandable medical device of FIG. 2;

FIG. 4 is an enlarged cross-section of an opening illustrating a therapeutic agent for directional delivery to a lumen of a blood vessel;

FIG. 5 is an enlarged cross-section of an opening illustrating a first therapeutic agent provided for delivery to a lumen of the blood vessel and a second therapeutic agent provided for delivery to a wall of the blood vessel; and

FIG. 6 is an enlarged cross-section of an opening illustrating first and second therapeutic agents for delivery to a lumen of the blood vessel.

### Detailed Description

The present invention relates to method and apparatus for treatment of acute ischemic syndromes including acute myocardial infarction. The methods and devices provide for delivery of therapeutic agents locally to the myocardial tissue to limit the necrotic zone in ischemic injury. The local delivery of the therapeutic agents avoid the need for systemic delivery and associated need to administer additional protective agents to prevent damage to non-target tissues.

First, the following terms, as used herein, shall have the following meanings:

The terms "drug" and "therapeutic agent" are used interchangeably to refer to any therapeutically active substance that is delivered to a bodily conduit of a living being to produce a desired, usually beneficial, effect.

The term "matrix" or "biocompatible matrix" are used interchangeably to refer to a medium or material that, upon implantation in a subject, does not elicit a detrimental response sufficient to result in the rejection of the matrix. The matrix typically does not provide any therapeutic responses itself, though the matrix may contain or surround a therapeutic agent, and/or modulate the release of the therapeutic agent into the body. A matrix is also a medium that may simply provide support, structural integrity or structural barriers. The matrix may be polymeric, non-polymeric, hydrophobic, hydrophilic, lipophilic, amphiphilic, and the like. The matrix may be bioresorbable or non-bioresorbable.

The term "bioresorbable" refers to a matrix, as defined herein, that can be broken down by either chemical or physical process, upon interaction with a physiological environment. The matrix can erode or dissolve. A bioresorbable matrix serves a temporary function in the body, such as drug delivery, and is then degraded or broken into components that are metabolizable or excretable, over a period of time from minutes to years, preferably less than one year, while maintaining any requisite structural integrity in that same time period.

The term "ischemia" refers to local hypoxia resulting from obstructed blood flow to an affected tissue.

The term "ischemic injury" as used herein refers to both injury due to obstructed blood flow and reperfusion injury caused by removal of the obstruction.

The term "openings" includes both through openings and recesses.

The term "pharmaceutically acceptable" refers to the characteristic of being non-toxic to a host or patient and suitable for maintaining the stability of a beneficial agent and allowing the delivery of the beneficial agent to target cells or tissue.

The term "polymer" refers to molecules formed from the chemical union of two or more repeating units, called monomers. Accordingly, included within the term "polymer" may be, for example, dimers, trimers and oligomers. The polymer may be synthetic, naturally-occurring or semisynthetic. In preferred form, the term "polymer" refers to molecules which typically have a M_{w} greater than about 3000 and preferably greater than about 10,000 and a M_{w} that is less than about 10 million, preferably less than about a million and more preferably less than about 200,000. Examples of polymers include but are not limited to, poly-a-hydroxy acid esters such as, polylactic acid (PLLA or DLPLA), polyglycolic acid, polylactic-co-glycolic acid (PLGA), polylactic acid-co-caprolactone; poly (block-ethylene oxide-block-lactide-co-glycolide) polymers (PEO-block-PLGA and PEO-block-PLGA-block-PEO); polyethylene glycol and polyethylene oxide, poly (block-ethylene oxide-block-propylene oxide-block-ethylene oxide); polyvinyl pyrrolidone; polyorthoesters; polysaccharides and polysaccharide derivatives such as polyhyaluronic acid, poly (glucose), polyalginic acid, chitin, chitosan, chitosan derivatives, cellulose, methyl cellulose, hydroxyethylcellulose, hydroxypropylcellulose, carboxymethylcellulose, cyclodextrins and substituted cyclodextrins, such as beta-cyclo dextrin sulfo butyl ethers; polypeptides, and proteins such as polylysine, polyglutamic acid, albumin; polyanhydrides; polyhydroxy alkonoates such as polyhydroxy valerate, polyhydroxy butyrate, and the like.

The term "primarily" with respect to directional delivery, refers to an amount greater than about 50% of the total amount of beneficial agent provided to a blood vessel.

The term "restenosis" refers to the renarrowing of an artery following an angioplasty procedure which may include stenosis following stent implantation.

### Methods for Locally Delivering Drugs to Preserve Myocardial Cell Viability

Implantable medical devices in the form of stents when implanted directly at or near a site of a previously occluded blood vessel can be used to deliver therapeutic agents to the myocardial tissue at and downstream of the implantation site. The delivery of the agent locally at the ischemic injury site improves the viability of the cells by reducing ischemic injury to the myocardial cells including reperfusion injury which may occur upon return of blood flow to the ischemic tissue. In cases where reperfusion therapy is performed by angioplasty, a stent is often delivered to the reopened occlusion site. A drug delivery stent for delivery of a therapeutic agent for treatment of ischemic injury can be implanted at the implantation site in the traditional manner after angioplasty. The drug delivery stent for delivery of the therapeutic agent implanted at or near the occlusion site following reperfusion therapy provides the advantage of reduction of ischemic injury including reduction of reperfusion injury without the difficulties associated with systemic delivery of the therapeutic agent.

The metabolic mechanisms of reperfusion injury are not completely clear. Lack of oxygen and accumulation of metabolic products change the energy transfer in the tissue. After reperfusion, the oxidation of glucose remains depressed, as does contractile function. In addition, reperfusion damage occurs due to the inflammatory response. Reperfused ischemic tissue attracts leukocytes which release proteolytic enzymes and oxidants that in turn promote further inflammation followed by eventual healing and scarring. Therefore, anti-inflammatory drugs that dampen the inflammatory response can reduce reperfusion injury. Protease inhibitors, antioxidants, vasodilators, and other cardio-protective agents can also improve tissue function following reperfusion. Vasodilators when delivered downstream of an occlusion, either acute or nonacute, can expand vessel dimensions and thus increase blood flow to an ischemic area.

The drugs which are particularly well suited for the reduction of ischemic injury following acute myocardial infarction or other ischemic injuries include, but are not limited to, vasodilators, such as adenosine, and dipyridamole; nitric oxide donors; prostaglandins and their derivatives; antioxidants; membrane stabilizing agents; anti-TNF compounds; anti-inflamatories including dexamethasone, aspirin, pirfenidone, meclofenamic acid, and tranilast; hypertension drugs including Beta blockers, ACE inhibitors, and calcium channel blockers; anti-metabolites, such as 2-CdA; vasoactive substances including vasoactive intestinal polypeptides (VIP); insulin; cell sensitizers to insulin including glitazones, P par agonists, and metformin; protein kinases; antisense oligonucleotides including resten-NG; immuno-suppressants including sirolimus, everolimus, tacrolimus, etoposide, and mitoxantrone; antithrombins; antiplatelet agents including tirofiban, eptifibatide, and abciximab; cardio protectants including, VIP, pituitary adenylate cyclase-activating peptide (PACAP), apoA-I milano, amlodipine, nicorandil, cilostaxone, and thienopyridine; anti-leukocytes; cyclooxygenase inhibitors including COX-1 and COX-2 inhibitors; and petidose inhibitors which increase glycolitic metabolism including omnipatrilat.

Agents for the treatment of ischemic injury may also be delivered using a gene therapy-based approach in combination with an expandable medical device. Gene therapy refers to the delivery of exogenous genes to a cell or tissue, thereby causing target cells to express the exogenous gene product. Genes are typically delivered by either mechanical or vector-mediated methods. Mechanical methods include, but are not limited to, direct DNA microinjection, ballistic DNA-particle delivery, liposome-mediated transfection, and receptor-mediated gene transfer. Vector-mediated delivery typically involves recombinant virus genomes, including but not limited to those of retroviruses, adenoviruses, adeno-associated viruses, herpesviruses, vaccinia viruses, picomaviruses, alphaviruses, and papovaviruses .

According to one aspect of the invention, a stent or other local delivery device is used for local delivery of insulin following acute MI and reperfusion. Insulin is a hormone which improves glycolic metabolism and ATP production. Insulin also may act as a vasodilator, an anti-inflammatory, and an antiplatelet agent. Thus, insulin acts by several mechanisms to decrease infarct size by reducing inflammation, slowing the rate of ischemic necrosis, decreasing circulating levels of FFA and myocardial FFA uptake, restoring myocardial glycogen stores and improving contractile function.

The insulin for use in the present invention can be human, non-human, or synthetic and can be complete or fragments. Preferably the insulin is a stable, short acting form which is resistant to radiation. Insulin in its crystalline form may be used for improved resistance to radiation. When the insulin is combined with a polymer an agent may be added to preserve bioactivity. Insulin has been found to retain its bioactivity for administration periods of at least 24 hours when delivered in poly(lactide-co-glycolide) (PLGA). For substantially longer administration periods, an antacid or other agent may be used to maintain a required pH for continued bioactivity from a PLGA matrix.

In one example, insulin can be combined with a hydrogel or proto-hydrogel matrix. The insulin/hydrogel is loaded into the openings of a stent and dehydrated. Rehydration of the hydrogel causes the hydrogel to swell and allows the insulin to be released from the hydrogel.

Although the delivery of insulin from a stent has been described herein primarily for delivery to the lumen of a blood vessel for reducing ischemic injury, insulin may also be delivered murally from the stent to treat restenosis.

According to another aspect of the invention, a stent or other local delivery device is used for local delivery of VIP following acute MI and reperfusion. VIP is a neuropeptide which is naturally released by the heart during coronary occlusion and exerts a protective effect on the heart. VIP acts as a vasodilator, a platelet inhibitor, and an antiproliferative. VIP acts by inhibiting the production of pro-inflammatory agents and stimulating the production of anti-inflammatory cytokines in activated macrophages.

In one embodiment of the invention, a drug which is suited for the reduction of ischemic injury is delivered at or near the site of a reopened occlusion following myocardial infarction or other acute ischemic syndromes. The delivery of the drug at or near the site of the previous occlusion allows the drug to be delivered by the blood flow downstream to the reperfused tissue. The drug can be delivered by a stent containing drug in openings in the stent as described further below. The drug can also be delivered by a drug coated stent, an implant, microspheres, a catheter, coils, or other local delivery means.

For example, microspheres, coils, lyposomes, or other small drug carriers can be delivered locally at or near the site of a previous occlusion with a catheter or drug delivery stent. These small drug carriers are released and pass downstream into the myocardium where they may implant themselves delivering the drug directly to the ischemic tissue.

The drug can be released over an administration period which is dependent on the mode of action of the drug delivered. For example, insulin may be delivered over an administration period of from a few minutes up to weeks, preferably insulin is delivered over a period of at least 1 hour, more preferably at least 2 hours, and more preferably about 10-48 hours. In another example, a fast acting vasodilator, such as adenosine or a derivative thereof, may be delivered over a shorter administration period of a few seconds to a few minutes.

In one example, the drug for reduction of ischemic injury is delivered from a stent primarily in a luminal direction with minimal drug being delivered directly from the stent in the direction of the vessel wall. This stent may be placed alone in the occlusion or may be placed in addition to another stent (bare stent or drug delivery stent) placed in connection with an angioplasty procedure. The stent for delivery of ischemic injury treatment agent may be placed within or adjacent another previously placed stent. The implantation site for the stent may be at or near the site of the occlusion. An implantation site may also be selected at or near a location of a plaque rupture site or a vessel narrowing.

The present invention is also particularly well suited for the delivery of a second therapeutic agent primarily from a mural side of a stent in addition to the first agent delivered primarily from the luminal side of the stent for reduction of ischemic injury. The primarily murally delivered agents may include antineoplastics, antiangiogenics, angiogenic factors, antirestenotics, anti-thrombotics, such as heparin, antiproliferatives, such as paclitaxel and Rapamycin and derivatives thereof.

In the dual agent example, a drug suited for the reduction of ischemic injury is delivered primarily luminally from a stent while a drug for the treatment of restenosis is delivered primarily murally from the stent. In one likely example, the first drug for the reduction of ischemic injury is delivered at a first delivery rate for a first administration period, such as over a period of about to about 24 hours, while the second drug for the treatment of restenosis is delivered at a second delivery rate for a second administration period, such as over a period of about 2 days or longer, preferably about 3 days or longer, and more preferably about 10 days or longer.

In another dual agent delivery example, two agents for treatment of ischemic injury are both delivered primarily luminally. The two agents may be delivered over different administration periods depending on the mode of action of the agents. For example, a fast acting agent may be delivered over a short period of a few minutes while a slower acting agent is delivered over several hours or days.

In another example, the local delivery of a therapeutic agent suited for the reduction of ischemic injury is used in combination with one or more systemically delivered therapeutic agents. For example, when insulin is delivered locally to the site of a previously occluded blood vessel, glucose and/or potassium can be delivered systemically if needed. However, a much smaller amount of systemically administered glucose and/or potassium will be needed than in the case of systemically administered insulin. In addition, glucose and/or potassium may be delivered locally by the same drug delivery stent as the insulin or by another local delivery vehicle, such as another stent, catheter, or implant.

Some of the therapeutic agents for use with the present invention which may be transmitted primarily luminally, primarily murally, or both include, but are not limited to, antiproliferatives, antithrombins, immunosuppressants, antilipid agents, anti-inflammatory agents, antineoplastics, antiplatelets, angiogenic agents, anti-angiogenic agents, vitamins, antimitotics, metalloproteinase inhibitors, NO donors, estradiols, anti-sclerosing agents, and vasoactive agents, endothelial growth factors, estrogen, beta blockers, AZ blockers, hormones, statins, insulin growth factors, antioxidants, membrane stabilizing agents, calcium antagonists, retenoid, bivalirudin, phenoxodiol, etoposide, ticlopidine, dipyridamole, and trapidil alone or in combinations with any therapeutic agent mentioned herein. Therapeutic agents also include peptides, lipoproteins, polypeptides, polynucleotides encoding polypeptides, lipids, protein-drugs, protein conjugate drugs, enzymes, oligonucleotides and their derivatives, ribozymes, other genetic material, cells, antisense, oligonucleotides, monoclonal antibodies, platelets, prions, viruses, bacteria, and eukaryotic cells such as endothelial cells, stem cells, ACE inhibitors, monocyte/macrophages or vascular smooth muscle cells to name but a few examples. The therapeutic agent may also be a pro-drug, which metabolizes into the desired drug when administered to a host. In addition, therapeutic agents may be pre-formulated as microcapsules, microspheres, microbubbles, liposomes, niosomes, emulsions, dispersions or the like before they are incorporated into the therapeutic layer. Therapeutic agents may also be radioactive isotopes or agents activated by some other form of energy such as light or ultrasonic energy, or by other circulating molecules that can be systemically administered. Therapeutic agents may perform multiple functions including modulating angiogenesis, restenosis, cell proliferation, thrombosis, platelet aggregation, clotting, and vasodilation. Anti-inflammatories include non-steroidal anti-inflammatories (NSAID), such as aryl acetic acid derivatives, e.g., Diclofenac; aryl propionic acid derivatives, e.g., Naproxen; and salicylic acid derivatives, e.g., aspirin, Diflunisal. Anti-inflammatories also include glucocoriticoids (steroids) such as dexamethasone, prednisolone, and triamcinolone. Anti-inflammatories may be used in combination with antiproliferatives to mitigate the reaction of the tissue to the antiproliferative.

Some of the agents described herein may be combined with additives which preserve their activity. For example additives including surfactants, antacids, antioxidants, and detergents may be used to minimize denaturation and aggregation of a protein drug, such as insulin. Anionic, cationic, or nonionic detergents may be used. Examples of nonionic additives include but are not limited to sugars including sorbitol, sucrose, trehalose; dextrans including dextran, carboxy methyl (CM) dextran, diethylamino ethyl (DEAE) dextran; sugar derivatives including D-glucosaminic acid, and D-glucose diethyl mercaptal; synthetic polyethers including polyethylene glycol (PEO) and polyvinyl pyrrolidone (PVP); carboxylic acids including D-lactic acid, glycolic acid, and propionic acid; detergents with affinity for hydrophobic interfaces including n-dodecyl-β-D-maltoside, n-octyl-β-D-glucoside, PEO-fatty acid esters (e.g. stearate (myrj 59) or oleate), PEO-sorbitan-fatty acid esters (e.g. Tween 80, PEO-20 sorbitan monooleate), sorbitan-fatty acid esters (e.g. SPAN 60, sorbitan monostearate), PEO-glyceryl-fatty acid esters; glyceryl fatty acid esters (e.g. glyceryl monostearate), PEO-hydrocarbon-ethers (e.g. PEO-10 oleyl ether; triton X-100; and Lubrol. Examples of ionic detergents include but are not limited to fatty acid salts including calcium stearate, magnesium stearate, and zinc stearate; phospholipids including lecithin and phosphatidyl choline; CM-PEG; cholic acid; sodium dodecyl sulfate (SDS); docusate (AOT); and taumocholic acid.

### Implantable Medical Devices with Openings

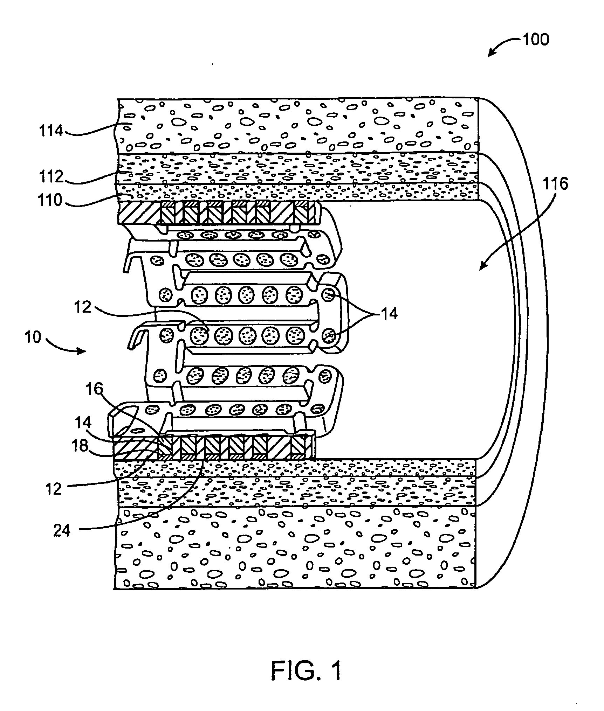

FIG. 1 illustrates an expandable medical device 10 in the form of a stent implanted in a lumen 116 of an artery 100. A wall of the artery 100 includes three distinct tissue layers, the intima 110, the media 112, and the adventitia 114. When the expandable medical device 10 is implanted in an artery at an occlusion site, a therapeutic agent delivered from the expandable medical device to the lumen 116 of the artery 100 is distributed locally to the tissue at the site of the occlusion and downstream by the blood flow.

One example of an expandable medical device 10, as shown in FIGS. 1-3, includes large, non-deforming struts 12, which can contain openings 14 without compromising the mechanical properties of the struts, or the device as a whole. The non-deforming struts 12 may be achieved by the use of ductile hinges 20 which are described in detail in U.S. Patent No. 6,241,762, which is incorporated herein by reference in its entirety. The openings 14 serve as large, protected reservoirs for delivering various beneficial agents to the device implantation site.

The relatively large, protected openings 14, as described above, make the expandable medical device of the present invention particularly suitable for delivering large amounts of therapeutic agents, larger molecules or genetic or cellular agents, and for directional delivery of agents. The large non-deforming openings 14 in the expandable device 10 form protected areas or receptors to facilitate the loading of such an agent, and to protect the agent from abrasion, extrusion, or other degradation during delivery and implantation.

FIG. 1 illustrates an expandable medical device for directional delivery of a therapeutic agent 16. The openings 14 contain the therapeutic agent 16 for delivery to the lumen 116 of the blood vessel and an optional barrier layer 18 in or adjacent the mural side of the openings.

The volume of beneficial agent that can be delivered using openings 14 is about 3 to 10 times greater than the volume of a 5 micron coating covering a stent with the same stent/vessel wall coverage ratio. This much larger beneficial agent capacity provides several advantages. The larger capacity can be used to deliver multi-drug combinations, each with independent release profiles, for improved efficacy. Also, larger capacity can be used to provide larger quantities of less aggressive drugs and to achieve clinical efficacy without the undesirable side-effects of more potent drugs, such as retarded healing of the endothelial layer.

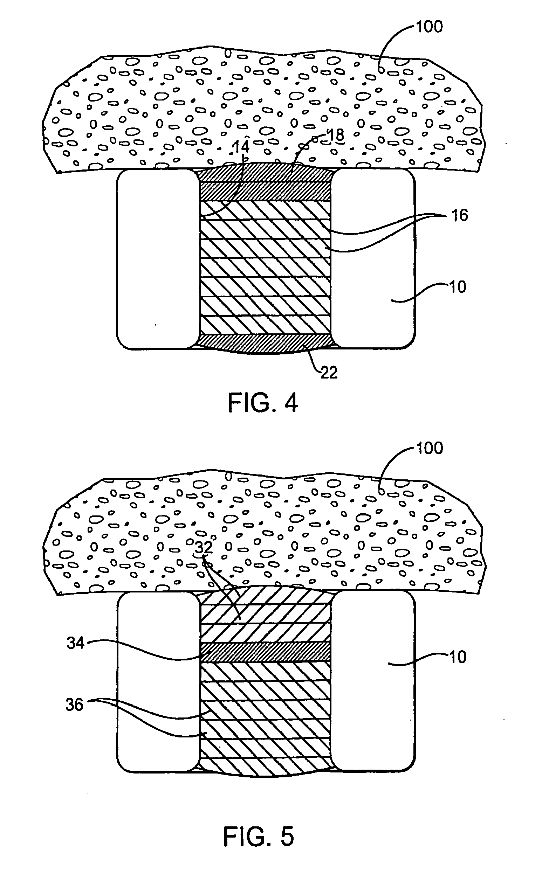

FIG. 4 shows a cross section of a portion of a medical device 10 in which one or more beneficial agents have been loaded into an opening 14 in multiple layers. Although multiple discrete layers are shown for ease of illustration, the layers may be discrete layers with independent compositions or blended to form a continuous polymer matrix and agent inlay. For example, the layers can be deposited separately in layers of a drug, polymer, solvent composition which are then blended together in the openings by the action of the solvent. The agent may be distributed within an inlay uniformly or in a concentration gradient. Examples of some methods of creating such layers and arrangements of layers are described in U.S. Patent Publication No. 2002/0082680, published on June 27, 2002, which is incorporated herein by reference in its entirety. The use of drugs in combination with polymers within the openings 14 allows the medical device 10 to be designed with drug release kinetics tailored to the specific drug delivery profile desired.

According to one example, the total depth of the opening 14 is about 50 to about 140 microns, and the typical layer thickness would be about 2 to about 50 microns, preferably about 12 microns. Each typical layer is thus individually about twice as thick as the typical coating applied to surface-coated stents. There can be at least two and preferably about five to twelve such layers in a typical opening, with a total beneficial agent thickness about 4 to 28 times greater than a typical surface coating. According to one embodiment of the present invention, the openings have an area of at least 5 x 10-6 square inches, and preferably at least 10 x 10-6 square inches.

In the example of FIG. 4, the mural side of the openings are provided with a barrier layer 18 which is a layer of polymer or other material having an erosion rate which is sufficiently slow to allow substantially all of the therapeutic agent in the therapeutic agent layers 16 to be delivered from the luminal side of the opening prior to complete erosion of the barrier layer. The barrier layer 18 prevents loss of the beneficial agent during transport, storage, and during the stent implantation procedure. However, the barrier layer 18 maybe omitted where mural and luminal delivery of the agent is acceptable.

In one example, the barrier layer 18 and/or the cap layer 22 may be formed by a material soluble in a different solvent from the therapeutic agent layers to prevent intermixing of layers. For example, where one or more layers of therapeutic agent and matrix have been deposited in the openings in a solvent (e.g. Insulin and PVP in water), it may be desirable to select a different polymer and solvent combination (e.g. PLGA in DMSO) for the barrier layer to prevent the therapeutic agent from mixing into the barrier layer. Another layer, such as a cap layer may be formed by a third non-mixing polymer and solvent combination (e.g. PLGA in anisole). In addition to the barrier layer and cap layer, other therapeutic agent layers, protective layers, or separating layers may also be formed of non-mixing polymer/solvent systems in this manner.

A cap layer 22 can be provided which serves as a seal during filling of the openings. The cap layer 22 is preferably a rapidly degrading biocompatible material.

Since each layer of both the barrier layer 18 and therapeutic agent 16 is created independently, individual chemical compositions and pharmacokinetic properties can be imparted to each layer. Numerous useful arrangements of such layers can be formed, some of which will be described below. Each of the layers may include one or more agents in the same or different proportions from layer to layer. Changes in the agent concentration between layers can be used to achieve a desired delivery profile. For example, a decreasing release of drug for about 24 hours can be achieved. In another example, an initial burst followed by a constant release for about one week can be achieved. Substantially constant release rates over time period from a few hours to months can be achieved. The layers may be solid, porous, or filled with other drugs or excipients.

FIG. 5 is a cross sectional view of a portion of an expandable medical device 10 including two or more therapeutic agents. Dual agent delivery systems such as that shown in FIG. 5 can deliver two or more therapeutic agents in different directions for the treatment of different conditions or stages of conditions. For example, a dual agent delivery system may deliver different agents in the luminal and mural directions for treatment of ischemia and restenosis from the same drug delivery device.

In FIG. 5, an antirestenotic agent 32 is provided at the mural side of the device 10 in one or more layers and a therapeutic agent 36 for reducing ischemic injury is provided at the luminal side of the device in one or more layers. A separating layer 34 can be provided between the agent layers. A separating layer 34 can be particularly useful when the administration periods for the two agents are substantially different and delivery of one of the agents will be entirely completed while the other agent continues to be delivered. The separating layer 34 can be any biocompatible material, which is preferably degradable at a rate which is equal to or longer than the longer of the administration periods of the two agents.

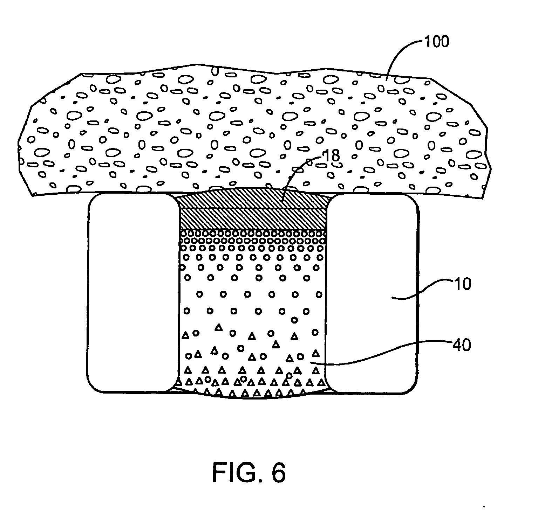

FIG. 6 illustrates an expandable medical device 10 including an inlay 40 formed of a biocompatible matrix with first and second agents provided in the matrix for delivery according to different agent delivery profiles. As shown in FIG. 6, a first drug illustrated by Os is provided in the matrix with a concentration gradient such that the concentration of the drug is highest adjacent the barrier layer 18 at the mural side of the opening and is lowest at the luminal side of the opening. The second drug illustrated by Δs is relatively concentrated in an area close to the luminal side of the opening. This configuration illustrated in FIG. 6 results in delivery of two different agents with different delivery profiles from the same inlay 40. The two different agents can be agents which treat ischemic injury by different modes of action, such as insulin and VIP.

In the embodiments described above, the therapeutic agent can be provided in the expandable medical device in a biocompatible matrix. The matrix can be bioerodible as those described below or can be a permanent part of the device from which the therapeutic agent diffuses. One or more barrier layers, separating layers, and cap layers can be used to separate therapeutic agents within the openings or to prevent the therapeutic agents from degradation or delivery prior to implantation of the medical device.

### EXAMPLES

### Example 1

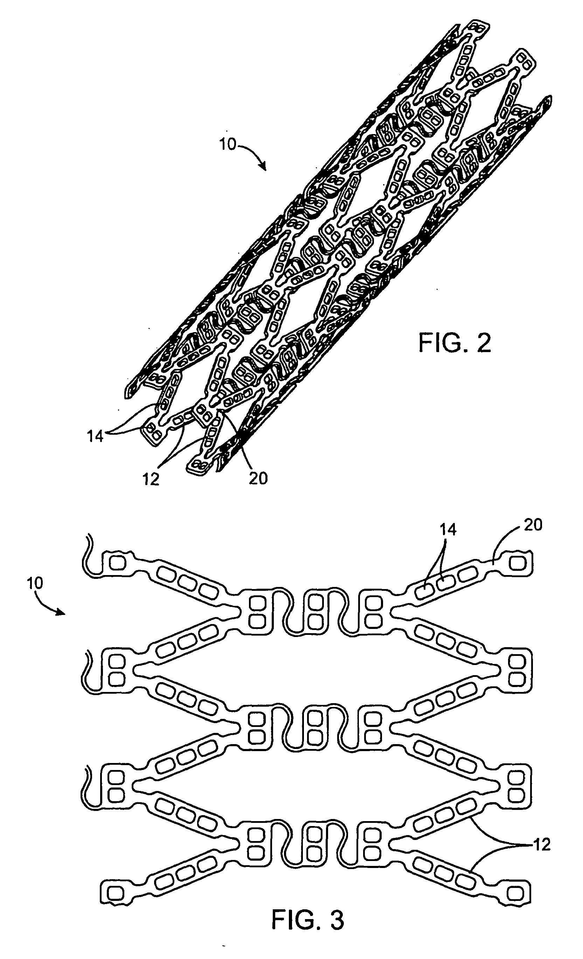

In this example, a drug delivery stent substantially equivalent to the stent illustrated in FIGS. 2 and 3 having an expanded size of about 3 mm X 17 mm is loaded with insulin in the following manner. The stent is positioned on a mandrel and an optional quick degrading layer is deposited into the openings in the stent. The quick degrading layer is low molecular weight PLGA provided on the luminal side to protect the subsequent layers during transport, storage, and delivery. The layers described herein are deposited in a dropwise manner and are delivered in liquid form by use of a suitable organic solvent, such as DMSO, NMP, or DMAc. A plurality of layers of insulin and low molecular weight PLGA matrix are then deposited into the openings to form an inlay of drug for the reduction of ischemic injury. The insulin and polymer matrix are combined and deposited in a manner to achieve a drug delivery profile which results in about 70% of the total drug released in about the first 2 hours, about 80% released in about 8 hours, and essentially 100% released in about 24 to about 48 hours. A barrier layer of moderate or high molecular weight PLGA, a slow degrading polymer, is deposited over the insulin layers to prevent the insulin from migrating to the mural side of the stent and the vessel walls. The degradation rate of the barrier layer is selected so that the cap layer does not degrade substantially until after the about 24-48 hour administration period.

The insulin dosage provided on the stent described is about 230 micrograms. The dosage has been calculated based on reported studies on systemic infusions of insulin which are estimated to deliver to the heart about 10 micrograms of insulin over a 24 hour period. The total dosage on the stent may range from about 5 micrograms to about 800 micrograms, preferably about 200 to about 400 micrograms.

### Example 2

In this example, a drug delivery stent substantially equivalent to the stent illustrated in FIGS. 2 and 3 having an expanded size of about 3 mm X 17 mm is loaded with insulin with a total dosage of about 230 micrograms in the following manner. The stent is positioned on a mandrel and an optional quick degrading layer is deposited into the openings in the stent. The quick degrading layer is PLGA. A plurality of layers of insulin and a poloxamer block copolymer of PEO and PPO (Pluronic F127) are then deposited into the openings to form an inlay of drug for the reduction of ischemic injury. The insulin and polymer matrix are combined at a ratio of about 33:67 and deposited in a manner to achieve a drug delivery profile similar to that described in Example 1. A barrier layer of high molecular weight PLGA, a slow degrading polymer, is deposited over the insulin layers to prevent the insulin from migrating to the mural side of the stent and the vessel walls. The degradation rate of the barrier layer is selected so that the cap layer does not degrade substantially until after the about 24-48 hour administration period.

### Examples 3

In this example, a drug delivery stent substantially equivalent to the stent illustrated in FIGS. 2 and 3 having an expanded size of about 3 mm X 17 mm is loaded with insulin with a total dosage of about 230 micrograms and with paclitaxel with a total dosage of about 10-30 micrograms in the following manner. The stent is positioned on a mandrel and an optional quick degrading layer is deposited into the openings in the stent. The quick degrading layer is PLGA. A plurality of layers of insulin and low molecular weight PLGA are then deposited into the openings to form an inlay of drug for the reduction of ischemic injury. The insulin and polymer matrix are combined and deposited in a manner to achieve a drug delivery profile similar to that described in Example 1. A plurality of layers of high molecular weight PLGA, a slow degrading polymer, and paclitaxel are deposited over the insulin layers to provide delivery of the paxlitaxel to the mural side of the stent and the vessel walls. The resorbtion rate of the paxlitaxel layers is selected so that these layers deliver paclitaxel continuously over an administration period of about 2 or more days.
Present application further discloses:
1. A method for reducing tissue damage following ischemic injury, the method comprising: identifying an implantation site within a blood vessel; delivering an expandable medical device containing a drug which preserves myocardial cell viability into the blood vessel to the selected implantation site; implanting the medical device at the implantation site; and locally delivering a therapeutic agent from the expandable medical device to tissue at the implantation site and to the blood vessels downstream of the implantation site over an administration period sufficient to reduce ischemic injury of the surrounding myocardial cells.
2. The method of 1., wherein the therapeutic agent is a vasodilator.
3. The method of 1., wherein the therapeutic agent is a hypertension drug.
4. The method of 1., wherein the therapeutic agent is a vasoactive substance.
5. The method of 1., wherein the therapeutic agent is a cardio protectant.
6. The method of 1., wherein the therapeutic agent is a membrane stabilizing agent.
7. The method of 1., wherein the therapeutic agent is an anti- inflammatory.
8. The method of 1., wherein the therapeutic agent is an antioxidant.
9. The method of 1., wherein the therapeutic agent is a membrane stabilizing agent.
10. The method of 1., wherein the therapeutic agent is insulin.
11. The method of 1., wherein the local delivery of the therapeutic agent comprises delivery of the therapeutic agent to reduce ischemic injury primarily from a luminal side of the medical device, and further comprising delivering an antiresenotic agent primarily from a mural side of the medical device.
12. A method of delivering insulin locally to myocardial tissue to reduce tissue damage following myocardial infarction and reperfusion, the method comprising : identifying an occlusion site within a blood vessel; treating the occlusion site to achieve reperfusion; and locally delivering insulin to the tissue at or near the treated occlusion site and downstream of the occlusion site to reduce ischemic injury.
13. The method of 12., wherein the insulin is delivered from a stent having a therapeutic dosage of insulin affixed thereto which is implanted at or near the occlusion site.
14. The method of 12., wherein the insulin is delivered by a catheter to the occlusion site.
15. The method of 12., wherein the insulin is delivered from one of spheres, coils, and implants located at or near the occlusion site.
16. The method of 12., wherein the insulin and a biocompatible polymer matrix are deposited within openings in an implantable medical device for local delivery to the occlusion site.
17. The method of 16., wherein the implantable medical device is a stent implanted at or near the occlusion site.
18. The method of 12., further comprising delivering the insulin from an implantable medical device implanted at or near the occlusion site and delivering an antirestenotic agent from the implantable medical device to the blood vessel wall at the occlusion site.
19. The method of 18., wherein the antirestenotic agent is paclitaxel.
   NB: Present application also discloses the above mentioned substances for use in the above mentioned methods for treatment by therapy. The present application further discloses:
20. An implantable medical device for delivering insulin locally to myocardial tissue, the device comprising: an implantable medical device configured to be implanted within a coronary artery; and a therapeutic dosage of insulin in a biocompatible polymer affixed to the implantable medical device, wherein the therapeutic dosage of insulin is released to the myocardial tissue at a therapeutic dosage and over an administration period effective to reduce ischemic injury of the myocardial tissue.
21. The device of 20., wherein the implantable medical device is a stent which is expandable within a coronary artery.
22. The device of 20., wherein the insulin and biocompatible polymer are deposited within openings in the implantable medical device.
23. The device of 20., wherein the insulin and biocompatible polymer are deposited on a surface of the implantable medical device.
24. The device of 20., wherein the administration period is about 1 hour or more.
25. The device of 20., wherein the administration period is about 10 to about 48 hours.
26. The device of 20., wherein the therapeutic dosage is about 5 to about 800 micrograms.
27. The device of 20., wherein the insulin and biocompatible polymer are deposited within openings in the implantable medical device and wherein a barrier layer is provided which substantially prevents delivery of the insulin to the artery wall.
28. The device of 20., further comprising an antirestenotic composition.
29. The device of 28., wherein the device is a substantially cylindrical device with a luminal side and a mural side.
30. The device of 29., wherein the insulin is deposited on the implantable medical device for delivery primarily to the luminal side of the device and the antirestenotic composition is deposited on the implantable medical device for delivery primarily to the mural side of the device.
31. The device of 20., wherein the insulin is selected from the group consisting of human insulin, non-human insulin, synthetic insulin.
32. The device of 20., wherein the therapeutic dosage of insulin is at least 200 micrograms.
33. An implantable medical device for delivering a therapeutic agent locally to myocardial tissue, the device comprising: an implantable medical device configured to be implanted within a coronary artery; and a therapeutic dosage of a therapeutic agent for treatment of ischemic injury following acute myocardial infarction, the therapeutic agent affixed to the implantable medical device in a manner such that the therapeutic agent is released to the myocardial tissue at a therapeutic dosage and over an administration period effective to reduce ischemic injury of the myocardial tissue.
34. The device of 33., wherein the therapeutic agent is a vasodilator.
35. The device of 33., wherein the therapeutic agent is a hypertension drug.
36. The device of 33., wherein the therapeutic agent is a vasoactive substance.
37. The device of 33., wherein the therapeutic agent is an cardio protectant.
38. The device of 33., wherein the therapeutic agent is a membrane stabilizing agent.
39. The device of 33., wherein the therapeutic agent is an anti- inflammatory.
40. The device of 33., wherein the therapeutic agent is an antioxidant.
41. The device of 33., wherein the therapeutic agent is a membrane stabilizing agent.
42. The device of 33., wherein the therapeutic agent is insulin.
43. The device of 33., wherein the therapeutic agent to reduce ischemic injury is affixed to the medical device for delivery primarily from a luminal side of the medical device, and further comprising an antiresenotic agent affixed to the medical device for delivery primarily from a mural side of the medical device.
44. The device of 33., wherein the implantable medical device is a stent which is expandable within a coronary artery.
45. The device of 33., wherein the therapeutic agent is deposited within openings in the implantable medical device.
46. The device of 33., wherein the therapeutic agent is affixed to the implantable medical device with a biocompatible polymer.
47. A stent for delivering insulin locally to myocardial tissue, the stent comprising: a substantially cylindrical expandable device body configured to be implanted within a blood vessel; and a therapeutic dosage of insulin in a biocompatible polymer affixed to the implantable medical device body.
48. The device of 47., wherein the insulin and biocompatible polymer are deposited within openings in the implantable medical device.
49. The device of 47., wherein the insulin and biocompatible polymer are deposited on a surface of the implantable medical device.
50. The device of 47., wherein the therapeutic dosage is configured for delivery over an administration period of about 1 hour or more.
51. The device of 47., wherein the therapeutic dosage is configured for delivery over an administration period of about 10 to about 48 hours.
52. The device of 47., wherein the therapeutic dosage is about 5 to about 800 micrograms.
53. The device of 47., wherein the insulin and biocompatible polymer are deposited within openings in the implantable medical device and wherein a barrier layer is provided which substantially prevents delivery of the insulin to the artery wall.
54. The device of 47., further comprising an antirestenotic composition.
55. The device of 54., wherein the insulin is deposited on the implantable medical device for delivery primarily to the luminal side of the device and the antirestenotic composition is deposited on the implantable medical device for delivery primarily to the mural side of the device.
56. The device of 47., wherein the insulin is selected from the group consisting of human insulin, non-human insulin, synthetic insulin.
57. The device of 47., wherein the therapeutic dosage of insulin is at least 5 micrograms.

## Claims

1. An ischemic-injury-reducing substance for use in a method for treatment of ischemic injury following AMI (acute myocardial infarction), the method comprising:
- identifying an implantation site within a blood vessel such as a coronary artery;
- implanting an expandable medical device within the blood vessel or coronary artery, wherein the substance is affixed to the expandable medical device; and
- releasing the substance to myocardial tissue primarily luminally at a therapeutic dosage and over an administration period effective to reduce ischemic injury of the myocardial tissue.

2. The substance of claim 1, being vasoactive, a vasodilator, or causing vessel expansion.

3. The substance of claim 2, being dipyridamole, or adenosine.

4. The substance of one of claims 1 to 3, wherein the substance is effective for reducing reperfusion injury.

5. The substance of claim 4, being an anti-inflammatory drug, a protease inhibitor, or an antioxidant.

6. The substance of one of claims 1 to 3, wherein the substance is effective for reducing injury due to obstructed blood flow.

7. The substance of claim 6, being a nitric oxide donor such as nicorandil, an anti-TNF agent, an anti-inflammatory drug or agent such as dexamethasone, aspirin, pifenidone, meclofenamic acid or tranilast, an immunosuppressant such as sirolimus or everolimus, a direct antithrombin or antiplatelet agent such as tirofiban, or a cyclooxygenase inhibitor such as COX-1 inhibitors or COX-2 inhibitors; or is an antioxidant or an insulin sensitizer such as PPAR agonists or metformin.

8. The substance of one of claims 1 to 7, wherein the method comprises delivering a faster acting agent over a shorter period and delivering a slower acting agent over a longer period.

9. The substance of claim 8, wherein the slower acting agent is delivered over several hours.

10. The substance of one of claims 1 to 9, wherein the method further comprises systemically delivering a therapeutic agent.

11. A stent for primarily luminal delivery of a drug effective for the reduction of ischemic injury, to a blood vessel over a period of time, wherein the stent contains said drug.

12. The stent of claim 11, wherein said drug is the substance of one of claims 1 to 10, in particular is one of claims 1 to 3.

13. The stent of claim 12, comprising a therapeutic dosage of a vasodilator in a biocompatible polymer affixed to the stent body, wherein the vasodilator and biocompatible polymer are deposited within the stent.

14. The stent of claim 13, wherein the therapeutic dosage is configured for delivery over an administration period of 1 hour or more, or 10 to 48 hours.

15. The stent of one of claims 11 to 14, being configured for dual agent delivery, wherein one or both of the agents are delivered primarily luminally.
